# EUROPEAN PATENT SPECIFICATION

(11) **EP 1 519 717 B1**
(45) Date of publication and mention of the grant of the patent: **29.09.2010**
(21) Application number: 03738316.3
(22) Date of filing: 04.07.2003
(51) Int. Cl.: A61K 9/50

(54) **CONTROLLED RELEASE COMPOSITION**
ZUSAMMENSETZUNG ZUR KONTROLLIERTEN WIRKSTOFFABGABE
COMPOSITION A LIBERATION CONTROLEE

(30) Priority: 05.07.2002 GB 0215656; 05.07.2002 GB 0215657
(43) Date of publication of application: 06.04.2005
(73) Proprietor: TEMREL LIMITED, Road Town, Tortola (VG)
(72) Inventor: SPEIRS, Christopher J., Leatherhead, Surrey KT22 9NG (GB); MOIR, Peter, Dungarvan, Country Waterford (IE); WILLIAMS, Richard, CH- 4052 Basel (CH); CLARK, Michael, Cheltenham, Gloustershire GL50 2EA (GB)
(74) Representative: Stones, James Alexander
(86) International application number: PCT/GB2003/002911
(87) International publication number: WO 2004/004696

(56) References cited:
- WO-A-97/02020
- US-A- 5 260 069
- US-A- 5 834 021
- US-A- 5 834 024
- US-B1- 6 267 990

## Description

The present invention relates to the use of polymethacrylate materials, especially those whose dissolution is pH dependent, and other coating materials whose dissolution is pH dependent, in the control of the release of an active compound in the intestinal tract. The present invention also relates to the use of prednisolone metasulphobenzoate (11,17-dihydroxy-21-[(3-sulphobenzoyl)oxy]pregna-1,4-diene-3-20-dione) and pharmacologically acceptable salts, especially the sodium salt, in the treatment of inflammatory bowel disease and especially Crohn's disease.

In particular, it provides a solid pharmaceutical composition having two or more pluralities of active compound containing particles coated with a desired thickness of a polymethacrylate material, or other pH dissolution dependent coating material, to control the release profile of the active compound such as prednisolone metasulphobenzoate. It also provides use of coating thickness of the polymethacrylate material, or other pH dissolution dependent coating material, to control the release profile of the active compound through the intestinal tract.

Unless it is clear from the context that the free ester is intended, the term "prednisolone metasulphobenzoate" is used herein to include pharmacologically acceptable salts of prednisolone metasulphobenzoate as well as the free ester.

It is desirable to be able to control the release of an active compound in the gastrointestinal tract. Some conditions require local treatment in the intestine and if drugs for that purpose are absorbed systemically, problematic side effects can occur. In other situations, the acidic conditions in the stomach can degrade some active compounds, especially peptides and proteins and a vehicle for their delivery to parts of the intestine from which they can be systemically absorbed or provide their therapeutic effect would be advantageous. Also, it may be advantageous for some active compounds, especially peptides and proteins, to be administered to specific sites in the intestinal tract for systemic absorption, which may be at two or more different locations. Examples are compounds whose systemic absorption depend upon locating M cells or Peyers patches.

In other situations, it is simply desirable that an active compound be administered to the patient continually over a set period of time in order to maintain a desired plasma concentration of the active and a controlled release oral composition provides a convenient and effective method of achieving this.

Some methods of controlling release of an active compound are known. For example, providing an enteric coating on a tablet or capsule in order to enable its passage beyond the stomach before degrading in the small intestine is well known. Also, it is known to administer an active compound to a patient in a slow release matrix. Another known method is to make a derivative of the active compound, for example a glucoronic acid derivative, which will not cleave until it comes into contact with an appropriate intestinal enzyme, for example glucoronidase, thereby releasing the active compound.

Of particular relevance to the provision of a controlled release formulation of active compounds are disorders of the intestinal tract, particularly those that would benefit from a local effect and a pertinent example is inflammatory bowel disease (IBD).

Inflammatory bowel disease covers chronic nonspecific inflammatory conditions of the gastrointestinal tract, of which the two major forms are Crohn's disease and ulcerative colitis.

Crohn's disease may affect any part of the gastrointestinal tract although it frequently affects the small intestine, especially the ileum and may also affect the jejunum and any part of the colon, including the rectum and especially the caecum. It is characterised by thickened areas of the gastrointestinal wall, with inflammation extending through all layers, deep ulceration and fissuring of the mucosa. The affected areas are often interspersed with areas of relatively normal tissue.

Sulphasalazine has been used to treat cases of Crohn's disease affecting the colon as has 5-aminosalicylic acid in an enteric coated or slow release form. Steroids are widely used to treat severe cases of inflammation of the colon, especially ulcerative colitis and Crohn's disease. Usually they are administered orally or parenterally to provide a systemic effect, or rectally by enema to provide a topical effect. Relatively high doses of steroids are required to treat severe cases of inflammatory bowel disease. However, systemic absorption produces serious side effects and although systemic absorption is lower with rectal administration, enemas treat only the lower colon and rectum and their use is inconvenient.

The most commonly used steroid in the oral treatment of inflammatory bowel disease is prednisolone (17,21-di-hydroxypregna-1,4-diene-3,11,20-trione) in the form of the free alcohol or an ester thereof, usually the acetate. Daily doses of 15 to 60 mg (calculated as the free alcohol) are required to treat severe cases of inflammatory bowel disease, but absorption at these doses is harmful. Accordingly, present treatment with prednisolone is limited in both dosage and duration of therapy.

Several methods and compositions for targeting or controlling the release of an active compound in the intestines have been proposed, often to treat inflammatory bowel disease and Crohn's disease.

US-A-4496553 relates to an oral pharmaceutical composition comprising 5-aminosalicylic acid (5-ASA) for the treatment of colitis ulcerosa or Crohn's disease. It discloses a slow-release tablet consisting of granules of 5-ASA coated with ethyl cellulose and compressed with microcrystalline cellulose granules, talc and sodium stearate. Tests with ileostomy patients showed that 50% of the active ingredient from the tablets is released in the small bowel. It states (in column 6, lines 15-22) that release can be controlled by varying one or more of the particle size of the granulated active ingredient, the thickness and permeability of the coating, the active ingredient proper and the pH conditions within the coated particle.

EP-B-0097651 discloses a composition for selectively administering 5-aminosalicylic acid to the large intestine, comprising a solid oral dosage form containing the active compound, with a coating of a 60 to 150 micrometer thick layer of an anionic polymer which is insoluble in gastric juice and in intestinal fluid below pH 7 but soluble in colonic intestinal juice, so that the dosage form remains intact until the colon.

EP-B-0572486 discloses an orally administrable pharmaceutical dosage form which comprises a plurality of granules of a drug, such as 5-aminosalicylic acid, coated with a material which dissolves in the intestine and contained within a capsule which is also coated with a material which dissolves in the intestine. The composition is for selectively administering the drug to the intestine. It is stated that the granules are preferably contained within an enterically coated capsule which releases the granules in the small intestine and that the granules are coated with a coating which remains substantially intact until they reach at least the ileum and preferably thereafter provide a sustained release of the drug through the colon.

EP-A-0772443 discloses a non-disintegratable solid enteric pharmaceutical composition comprising prednisolone metasulphobenzoate having relatively rapid dissolution at pH 6.5 from an excipient matrix, and dosage forms containing pellets of the composition. The rapid dissolution is increased by the presence of a rheological modifying super-disintegrant in an amount of at least 5% by weight, but insufficient to cause disintegration of the composition. It is stated that the composition may comprise a plurality of such pellets, which may be coated in an enteric coating such as cellulose acetate phthalate or, preferably, partly methyl esterified methacrylic acid polymers having a ratio of free acid groups to ester groups of about 1:2, contained in a capsule that is enterically coated with a suitable coating material. The coating material on the pellets is preferably one that is insoluble in gastric juices and intestinal fluid below pH 7, but is soluble in lower intestinal fluid. The enteric coating material of the capsule is chosen to protect the capsule during passage through the stomach. The composition is intended for use in the treatment of Crohn's disease.

EP-B-0502032 discloses a formulation for site specific release of an active compound in the colon for the treatment of diseases of the colon such as ulcerative colitis and Crohn's disease. The active may be, for example, prednisolone or 5-aminosalicylic acid among others. The formulation comprises an active compound and amorphous amylose with an outer coating of cellulose or an acrylic polymer material. The active compound is preferably coated with glassy amylose, which tends not to degrade until it reaches the colon where it is attacked by amylose cleaving enzymes provided by microbial flora normally present in the colon. The composition is further coated with a cellulose or acrylic polymer material, which enhances the delayed release property of the amylose coated composition. The rate of release of the active compound from the composition once it reaches the colon may be controlled by varying the thickness of inner amylose coating provided. It states that it is also possible to vary the release in the colon by coating different particles of the active compound with amylose of different thicknesses. Release characteristics can be further varied by drying, which affects pore size and permeability or by adding a fatty or waxy substance to retard penetration of water. It is preferred that the cellulose or acrylic polymer outer coating material displays pH independent degradation.

WO-A-9921536 relates to a controlled release composition suitable for delivery of an active ingredient to the colon. It discloses a composition which comprises 5-aminosalicylic acid spheres also containing microcrystalline cellulose and having diameters in the range 1.00 to 1.40 mm, which spheres are coated with a mixed solvent (water and an organic water miscible solvent) amylose/ethyl cellulose composition, although the latter may instead be an acrylic polymer. The release profiles were examined for a range of amylose/ethyl cellulose ratios and coating thicknesses. It was found that coatings with a high proportion of ethyl cellulose resulted in very little drug release due to the absence of continuous amylose channels through the coat surface to the core of the pellet, whereas a coating with a high proportion of amylose resulted in films whose structure was compromised. Accordingly, where higher amylose concentrations were present in the coatings, a thicker coating was applied and the results showed that in such circumstances release of the active compound should not take place before the colon.

EP-A-0264989 discloses sustained release pharmaceutical formulations of rhein derivatives. In particular, it describes formulations intended to provide hematic levels of the drug for up to 24 hours from administration. As can be seen from Example 2 of this reference, the general concept of coating particles with different thicknesses of a material (cellulose acetophthalate, in the Example) in order to release the drug compound at different rates so as to provide sustained release over a predetermined time period is disclosed.

US-A-5529790 discloses pharmaceutical formulations which provide delayed and sustained release of a drug from the formulation by means of a hydratable diffusion barrier coating. The delay is a consequence of the rate of hydration and the thickness of the coating and the sustained release results from the permeability and thickness of the coating. The diffusion barrier preferably consists of a film-forming material that is insoluble in intestinal conditions and at least one further additive which controls the rate of hydration and permeability of the diffusion barrier. The preferred film-forming polymers are aqueous dispersions of fully esterified acrylic resins (e.g. Eudragit NE30D), fully esterified acrylic resins containing quaternary amine side chains (e.g. Eudragit RS30D) or aqueous dispersions of ethyl cellulose. A preferred additive for controlling the rate of hydration and the permability is magnesium stearate. The drug (e.g. diltiazem hydrochloride) may be formulated as spherical microparticles having a diameter in the range 500-1500 µm and is preferably formulated in two batches of particles, a long delay batch having a low rate of hydration and low permeability and a short delay batch having a relatively high rate of hydration and a high permeability, so that sustained release of the drug can be effected over an extended period of time. Dissolution studies were carried out on particles having different coating thicknesses.

US-A-4728512 discloses a pharmaceutical formulation comprising three groups of drug-releasing pellets presented in, for example, a capsule, of which each group of pellets releases the drug at a different time in the patient's digestive system. In particular, it discloses a formulation where one group of pellets is uncoated and releases the drug immediately upon release of the pellets from the capsule, a second group of pellets which have a pH-dependent coating (e.g. 20-30 wt% Eudragit S) and a third group of pellets which have a pH-independent coating, such as a dual-coat system where a time-dependent undercoat (e.g. hydroxypropyl methyl cellulose) is further coated with a hydratable diffusion barrier coating (e.g. Eudragit E30D and metallic stearate). The formulation thereby consists of three drug:release systems which provide drug release maximums during the periods 0-2 hours from administration, 2-6: hours from administration and 4-10 hours from administration respectively. The formulation provides three doses of drug over a period of, for example. 12 hours, by releasing the drug on three occasions in an amount according to the relative quantity of each group of particles. The groups of particles are coated with different thicknesses of coating materials and therefore the document discloses the general concept of using different groups of particles with different release properties to release the active compound at different locations in the intestinal tract (by virtue of the different delay in releasing the drug from the second and third groups of pellets) .

Both US-A-5260069 and US-A-5834024 disclose pharmaceutical compositions comprising at least two pluralities of particles. The pluralities may be coated with different thicknesses of a coating material comprising a polymer blend. The blend comprises, as a major component, at least one water insoluble polymer and, as a minor component, a polymer whose solubility is dependent on pH. US-A-5260069 exemplifies compositions in which nifedipine and zidovudine are active components and US-A-5834024 exemplifies the use of diltiazem as the active component.

US-B-6267990 discloses a pharmaceutical composition comprising three pluralities of particles, one of which is uncoated and the other two are coated with different thicknesses of a pH dependent release coating material. The coated particles have a film undercoat (e.g. OPADRY^{™} II) which provides a smooth and even surface on to which the pH dependent release coating material is applied. US-B-6267990 exemplifies the use of the ACE inhibitor, captopril, as the active component.

US-A-5834021 exemplifies a pharmaceutical composition comprising a plurality of pellets comprising prednisolone metasulphobenzoate. The pellets are coated with a first pH dependent release coating material and then filled into a capsule which is then itself coated with a second pH dependent release coating material.

There is as yet no effective method or composition for controlling release of active compounds in the intestine, which overcomes or accounts for the variation in pH and the rate of transit that occurs throughout the intestinal tract.

An improved method and composition for controlling release of an active compound such as prednisolone metasulphobenzoate to the intestinal tract would be desirable.

The inventors have now found that employing a pH dissolution dependent polymethacrylate material at different thicknesses on particles of prednisolone metasulphobenzoate surprisingly results in release of prednisolone metasulphobenzoate at different rates at the same pH and in a controllable manner over a range of pH values. The thickness of the polymethacrylate coating employed may be chosen, depending upon the pH and the desired rate and location of release, to provide a controlled release profile of prednisolone metasulphobenzoate, pH dissolution dependent coating materials such as polymethacrylates are usually employed to provide release of an active compound at a single location in the intestinal tract. To the best of our knowledge and belief, the use of different coating thickness of pH dissolution dependent coating materials have not been used to provide continual or sustained release.

The invention concerns an oral pharmaceutical composition comprising two or more pluralities of pellets, said pellets comprising prednisolone metasulphobenzoate, wherein the pellets of each said plurality are coated with a different thickness of a polymethacrylate material to those of the or each other plurality, whereby prednisolone metasulphobenzoate is released at different locations in the intestinal tract.

The inventors have also found that application of this technology surprisingly may be extended to compositions comprising other active compounds.

Accordingly, in the first aspect of the present invention there is provided an oral pharmaceutical composition comprising two or more pluralities of pellets, said pellets comprising an active compound, wherein the pellets of each said plurality are coated with a different thickness, as determined by theoretical weight gain on coating, of a pH dissolution dependent coating material to those of the or each other plurality, wherein said coating material is applied on to the surface of the pellets and whereby the active compound is released at different locations in the intestinal tract.

Without wishing to be bound by any particular theory, the inventors believe that the graded release of the active compound from the compositions might be due to altered permeability of the coating rather than breakdown of the different thickness of coating. Observations indicate that the active compound would appear to permeate out of the composition before disintegration of the composition occurs.

The following features may be applied to either the prednisolone metasulphobenzoate embodiment of the first aspect of the present invention or to the more general embodiment.

The coating material may be any pH dissolution dependent coating material that is used or is useful in the coating of oral pharmaceutical dosage forms for delivery of an active compound to the intestine and is preferably a pH dissolution dependent cellulose derivative, such as cellulose acetate phthalate and hydroxypropyl methylcellulose acetate phthalate, or a polymethacrylate material, which is pH dissolution dependent.

The cellulose derivative is preferably selected from cellulose acetate phthalate, hydroxypropyl methylcellulose, hydroxypropyl methylcellulose phthalate, hydroxypropyl methylcellulose acetate phthalate and other single or multiple ester and/or ether derivatives of cellulose whose dissolution is pH dependent.

By pH dissolution dependent coating material, it is meant to include those materials that, according to the current state of the art, are insoluble in gastric media until a certain pH is reached and those that give pH dependent release of a drug when used as a coating material on oral pharmaceutical dosage forms.

The polymethacrylates which find particular utility in the present invention are anionic polymers of dimethylaminoethylmethacrylates, methacrylic acid and methacrylic acid esters in varying ratios.

The polymethacrylates may be copolymers of acrylic acids (such as methacrylic acid) and acrylic acid esters (such as methyl methacrylate or ethyl ethacrylate). Preferably, the polymethacrylates used in accordance with the present invention are methacrylic acid copolymers, which are based upon methacrylic acid and various acrylic acid esters (such as ethyl acrylate or methyl methacrylate) or mixtures thereof. More preferably, one or more copolymers of methacrylic acid and methyl methacrylate, preferably having a ratio of free carboxyl groups to ester groups of, for example, about 1:2 (sold under the registered Trade Mark EUDRAGIT S by Röhm Pharma GmbH of Darmstadt, Germany) and having a molecular weight of 135,000 or about 1:1 (available from Röhm Pharma GmbH under the registered Trade Mark EUDRAGIT L) or a mixture thereof is used.

Preferably, the present invention utilises those polymethacrylates whose dissolution is pH-dependent. By polymethacrylates whose dissolution is pH-dependent, it is meant to include those polymethacrylates that, according to the current state of the art, are insoluble in gastric media until a certain pH is reached and those that give pH dependent release of a drug when used as a coating material, for example see The Handbook of Pharmaceutical Excipients, 3rd Edn., Edited by Arthur H. Kibbe (American Pharmaceutical Society and Pharmaceutical Press, 2000). Preferably, the polymethacrylate material comprises a polymethacrylate that is insoluble in gastric media until a certain pH is reached and/or gives pH dependent release of a drug when used as a coating material, according to The Handbook of Pharmaceutical Excipients whose monograph thereon on pages 401-406 is incorporated herein by reference.

Such polymethacrylates include copolymers of methacrylic acid and methyl methacrylate having a ratio of free carboxyl groups to ester groups of about 1:2 (available as EUDRAGIT S from Röhm Pharma GmbH) or about 1:1 (available as EUDRAGIT L from Röhm Pharma GmbH) and a copolymer of methacrylic acid and ethyl acrylate having a ratio of free carboxyl groups to ester groups of about 1:1 (available under the registered Trade Mark EUDRAGIT L 30 D-55 or EUDRAGIT L 100-55 from Röhm Pharma GmbH). More preferably, the polymethacrylate is one that is soluble at a pH greater than 5.5 and still more preferably at greater than 6.

Preferably, the coating material coating the pellets of each plurality of pellets is the same as that coating those of the or each other plurality of pellets.

In one embodiment of the invention, the pellets of each of the pluralities may be coated with a different thickness of the coating material chosen at increments to provide a homogeneous release profile of the active compound along at least one selected portion of the intestinal tract or along the entire intestinal tract. The selected portion may be around, but preferably before and after, the ileo-caecal valve.

Preferably, the thickness of the coating material and the incremental differences are chosen to provide multi-site release of the active compound such that release is homogeneous through the intestine. It may be desirable, for example when administering an active compound for the treatment of Crohn's disease, to provide homogeneous release of the active compound along the ileum and the colon and more particularly the ascending colon.

In this embodiment, the invention may provide homogeneous release of the active compound that has the advantage over conventional sustained release preparations in that the incremental differences in thickness of the coating material, especially a polymethacrylate material, can be chosen to overcome the variations in pH and the varied rate of progression or transit of a capsule or tablet through the intestine.

In conventional sustained release preparations, the variation in the rate of progression through the intestinal tract may result in delivery of the active compound to certain parts of the intestine at a lower concentration than to other parts. Similarly, the variation in pH in different parts of the intestine tends to result in different rates of release from conventional sustained release preparations. This may result in a loss of effect.

In patients with inflammatory bowel disease, especially with active inflammation, the rate of transit through the intestine and the pH within the intestine are often abnormal. Conventional sustained release formulations which provide release of the active agent in a time or pH-dependent manner may not provide a predictable or effective delivery of the active agent to the target areas of the intestine. Such formulations can result in underdosing at certain sites or overdosing, "dose-dumping", at other sites.

In the present embodiment, such variations can be accounted for by, for example, coating pellets of each plurality of pellets with a chosen thickness of the coating material to provide multi-site release throughout the intestine, wherein the incremental differences in coating thickness between each plurality may vary. For example, in order to obtain homogeneous release to parts of the intestine through which there is a greater rate of passage and to parts with a lesser rate of passage, the incremental differences in coating thickness for the pluralities of pellets being delivered to the part of the intestine with a greater rate of passage will be smaller than to that with a lesser rate of passage, and/or the number of pellets in the plurality of pellets delivered to the part with greater rate of passage will be larger. Similarly, in order to provide homogeneous release to parts of the intestine with higher pH and with lower pH, a thicker coating should be provided on the pellets that are intended to release the active compound to the part of the intestine with the higher pH, although this will depend upon its location within the intestine. In this way, the rate of release of the active compound may be controlled in relation to variations in pH or transit through the intestine, without being solely dependent upon either a specific pH being reached or a specific time having elapsed before release of the active compound.

Alternatively, the coating thickness on each of the pluralities may be chosen to effect release of the active compound at specified locations of the intestine. For example, each of the pluralities of pellets may be coated with a different thickness of a coating material, whereby the active compound is released, for example, at locations around, but preferably before and after, the ileo-caecal valve.

Preferably, there are two pluralities of pellets; one plurality in which the pellets are coated with a thickness of the coating material so as to release the active compound at the distal ileum before the ileo-caecal valve and the other plurality in which the pellets are coated with a different thickness of the coating material so as to release the active compound at the proximal caecum, after the ileo-caecal valve. Preferably the coating material is a pH dissolution dependent polymethacrylate material, more preferably a methacrylic acid copolymer, and still more preferably a copolymer of methacrylic acid and methyl methacrylate, preferably having a ratio of free carboxyl groups to ester groups of about 1:2.

The coating on the pellets may be of a thickness corresponding to a theoretical weight gain on coating of 15% for one of the pluralities and 20% weight gain for the other and preferably the number of pellets in each plurality are present as a ratio of 15% weight gain coated pellets to 20% weight gain coated pellets of 1:3.

In order to further control the release profile of the active compound through the intestine, pellets from one plurality of pellets may be coated with a different coating material to those of another plurality of pellets. Pellets of one plurality may also be of a different size to those of another plurality.

The present invention can be utilised, for example, to administer active compounds which have a therapeutic effect locally in the intestine, to administer active compounds of a high molecular weight for local or systemic action and for the administration of any active compound for which controlled release through the intestinal tract would be of benefit, for example, active compounds whose systemic absorption depends upon location and rate of release in the intestine.

It is of particular utility to the provision of active compounds for local action at one or more sites in the intestinal tract. For example, in the treatment of inflammatory bowel disease and, in particular, Crohn's disease, where affected areas may be at various locations in the intestinal tract and the controlled delivery of an active compound to those areas without administering to unaffected areas minimises systemic absorption of the active compound and consequently any side effect that may result from systemic uptake.

In the administration of high molecular weight compounds, for example proteins or peptides, the present invention may be utilised to protect the active compound from degrading in the acidic conditions of the stomach and may, for example, provide delivery of the compound to areas of the intestine from which they may be absorbed or at which are located appropriate M-cells or Peyers patches.

The invention is particularly applicable to the delivery of high molecular weight compounds in which the integrity of the tertiary structure is critical to the efficacy and safety of the compound. A particular advantage of the present invention is that the oral pharmaceutical composition may be prepared under gentle conditions relative to most pharmaceutical processes, whilst providing a desired release profile of the compound in the intestinal tract.

An example of a high molecular weight compound, which would benefit from formulation in a composition of the present invention is erythropoietin, a glycosylated protein hormone and haematopoietic growth factor, which is considered useful in the management of anaemia in chronic renal failure among other conditions and has been investigated in the treatment of anaemia of inflammatory bowel disease as well as other normocytic-normochromic anaemias. Erythropoietin is conventionally administered subcutaneously or intravenously, although a tabletted form or erythropoietin has been disclosed (RU-A-2152206).

Other classes of high molecular weight compound which may benefit from the present invention include interferons, TNF antagonists and specific protein and polypeptide agonists and antagonists of the immune system, hormones, such as human growth hormone and cytokines and cytokine antagonists.

Other compounds and classes of compound whose administration may benefit from the present invention include analgesics and antipyretics; antibacterial and antiprotozoal agents, such as metronidazole and other nitroimidazole antibiotics and antibiotics active against anaerobic bacteria; clarithromycin and other macrolide antibiotics; gentamycin, ciprofloxacin, rifabutin and other such antibiotics active against infective organisms commonly associated with or causing disorders of the intestine; antiinflammatory agents such as, salicylates, for example 5-aminosalicylic acid, 4-aminosalicylic acid and derivatives, such as balsalazide, steroids, especially prednisolone metasulphobenzoate; probiotics and prebiotics which have been shown to influence the symptoms of inflammatory bowel disease and irritable bowel syndrome and recovery from antibiotic-associated diarrhoea; and pharmacologically active drug substances known to influence the symptoms of irritable bowel syndrome, for example those affecting the serotinergic system and those active at the site of opiate receptors. α-amylase and paracetamol may also be administered using the composition of the present invention.

Other compounds which may benefit from the present invention include certain compounds that have toxic effects which limit their clinical usefulness, especially by causing local toxicity in specific areas of the gastrointestinal tract. Included among such compounds are examples of antibiotics, bisphosphonates and antiinflammatory drugs. A particular example is metformin, which is intolerable to many patients due to adverse effects on the gastrointestinal tract. The present invention may be utilised to minimise the concentration of the compound at the specific sites of toxicity and so allowing an effective therapeutic dose to be administered with a reduction in adverse events.

The preferred compounds for use in the present invention are prednisolone metasulphobenzoate, 5-aminosalicylic acid, metronidazole, clarithromycin, metformin and erythropoietin.

In a preferred embodiment of the present invention, the composition further comprises a capsule, preferably an enterically coated capsule, within which the pluralities of pellets are contained. The capsule will usually be a soft, or preferably, hard gelatine capsule, although other capsules which will dissolve in the small intestine may be used. The enteric coating will protect the capsule during its passage through the stomach. Any suitable enteric coating material which is soluble in the small intestine can be used. For example, cellulose acetate phthalate, hydroxypropyl methylcellulose phthalate or initially ethyl cellulose followed by polyvinyl acetate phthalate may be used, but it is preferred to use an anionic polymer having an appropriate dissolution profile. The presently preferred polymers are anionic carboxylic, that is polymers in which the anionic groups are at least predominantly free carboxylic and/or esterified carboxylic groups. It is particularly preferred that the polymers should be acrylic polymers and the presently preferred polymers are copolymers of methacrylic acid and methyl methacrylate in which the ratio of free acid groups to ester groups is about 1:1 (i.e. Eudragit L).

Alternatively, the pellets may be compressed into a tablet, which may be enterically coated.

The capsule (or other dosage form) coating can and usually will contain plasticiser and possibly other coating additives such as colouring agents, gloss producers, talc and/or magnesium stearate as well known in the coating art. In particular, anionic carboxylic acrylic polymer coatings usually contain 10 to 25% by weight of a plasticiser, especially diethyl phthalate.

In a second aspect of the invention there is provided the use of the coating thickness of a pH dissolution dependent coating material on pellets comprising an active compound, wherein the coating material is applied on to the surface of the pellets, to control the release profile of the active compound in the intestinal tract. By pH dissolution dependent coating material, it is meant coating materials whose dissolution is dependent upon pH. For example, a polymethacrylate material which is insoluble at pH 2, but substantially soluble at greater than pH 5.5 is a pH dissolution dependent polymethacrylate material.

In a third aspect of the present invention, there is provided use of a pH dissolution dependent coating material in the preparation of a medicament as described above for the treatment of disorders of the intestinal tract. Typically, the medicament will be for use in the treatment of Crohn's disease.

In a fourth aspect of the present invention, there is provided a method of treating a disorder of the intestinal tract of a patient, said method comprising administering to a patient an effective amount of an active compound for treating that disorder in at least two pluralities of pellets each coated with a different thickness of a pH dissolution dependent coating material to release the active compound at locations in the intestinal tract at which symptoms of the disorder are displayed and/or at which receptors substrate for the active compound are located.

The disorder may be any disorder of the intestinal tract and the active compound may be any compound effective in treating that disorder, but preferably the disorder is any disorder mentioned above and preferably also the active compound is any of the active compounds mentioned above for treating the respective disorder. Most preferably, the disorder is inflammatory bowel disease, especially Crohn's disease or anaemia associated with irritable bowel disease and more preferably still, the active compound is prednisolone metasulphobenzoate, 5-aminosalicylic acid, metronidazole, clarithromycin, metformin orerythropoetin.

Antibiotics effective in the treatment of inflammatory bowel disease or infective disorders of the intestine are frequently toxic when absorbed and the present invention may be applied to administer them to their sites of action in the intestine, achieving sufficient local concentrations whilst minimising systemic uptake. Of particular application to the present invention are toxic antibiotics, such as gentamycin, particularly in patients predisposed to the toxic effects of such drugs such as those with renal dysfunction. Patients with chronic disorders of the intestine, for example Crohn's disease and pouchitis, requiring continued administration of certain antibiotics, for example, metronidazole, over long periods are likely to benefit particularly from the present invention.

Other possible actives include cytotoxic agents such as cyclophosphamide, cisplatin and other platinum drugs and vincristine and other vinca alkaloids; immunomodulators such as methotrexate, azathioprine and cyclosporin; and anti-parasitic agents such as albendazole.

The particles used in the present invention are pellets.

The pellets according to the present invention may be pellets having a diameter in the range 500 to 2500 µm, preferably 800 to 1700 µm, more preferably 800 to 1500 µm and still more preferably 1000-1500 µm. However, it should be appreciated that pellets may have a diameter anywhere within the aforementioned ranges, or outwith, and that a single dosage form according to the present invention may have particles of one or more diameter or range of diameters.

It should be appreciated that the actual coating thickness for any particular weight gain of coating depends upon the size and weight of the pellets.

Preferably the coating thickness according to the present invention is in the range 5% to 30%, more preferably 10% to 25% and most preferably about 15% and about 20%.

Dosage forms in accordance with the present invention may contain pellets which contain different active compounds. For example, one plurality of pellets may contain a first active compound and another plurality of pellets may contain a second, different, active compound. The pellets may be coated to provide different release profiles for each of the active compounds in the dosage form. The coating for each of the pluralities of pellets will typically be polymethacrylate materials of a composition and thickness to provide the desired release profile for each of the active compounds. Alternatively, one plurality of pellets may be coated with a different coating material from the other plurality, in order to take advantage of a differing release characteristic of a coating material other than a polymethacrylate.

The pluralities of pellets in any such dosage form will typically be administered in an enterically coated capsule.

The invention will now be illustrated by the following non-limiting Examples with reference to the accompanying Figures.

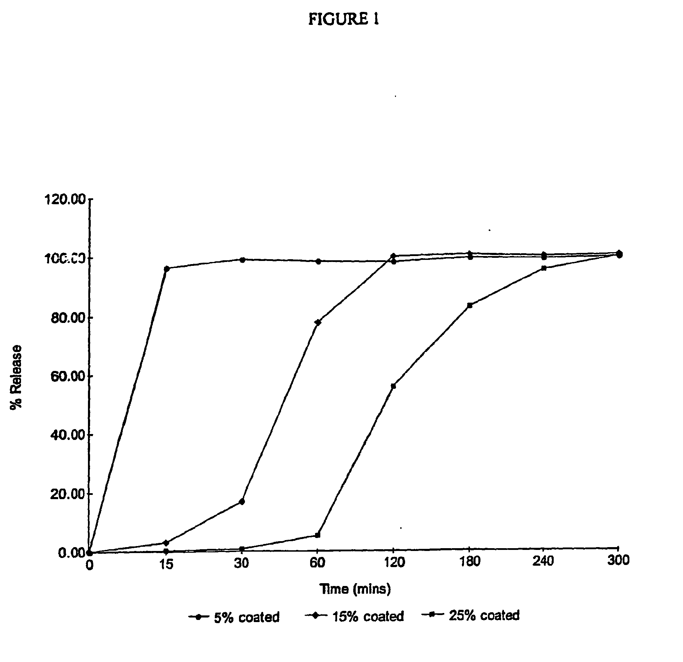

Figure 1 is a graph of percentage release (% Release), of prednisolone metasulphobenzoate from pellets coated with a methacrylic acid copolymer of methacrylic acid and methyl methacrylate having a ratio of free carboxyl groups to ester groups of 1:2 to a theoretical weight gain of 5%, 15% and 25%, against time;

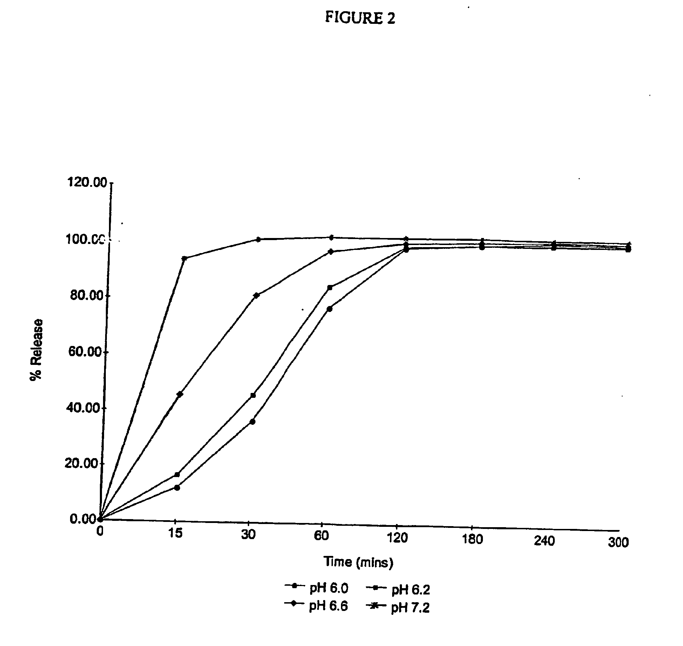

Figure 2 is a graph of percentage release (% Release), of prednisolone metasulphobenzoate from pellets coated with a methacrylic acid copolymer of methacrylic acid and methyl methacrylate having a ratio of free carboxyl groups to ester groups of 1:2 to a theoretical weight gain of 15%, against time at a pH of 6.0, 6.2, 6.6 and 7.2;

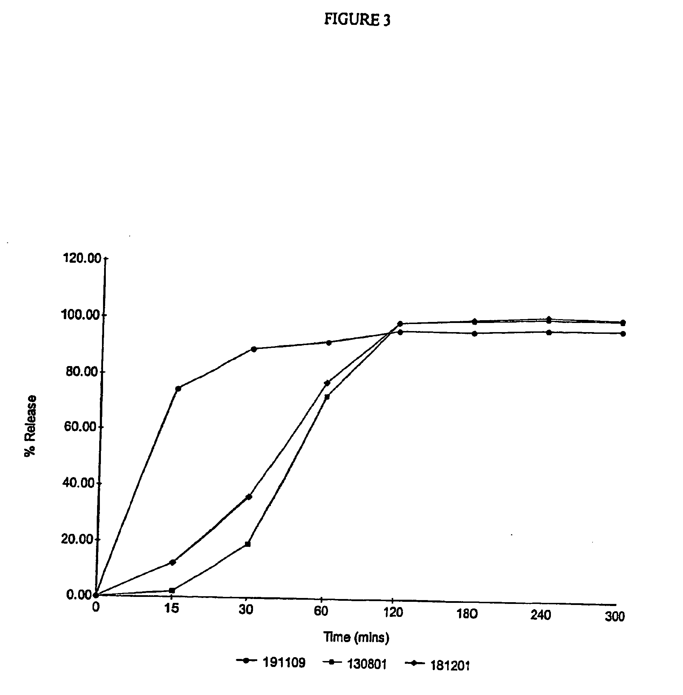

Figure 3 is a graph of percentage release (% Release), of prednisolone metasulphobenzoate from pellets coated with a methacrylic acid copolymer of methacrylic acid and methyl methacrylate having a ratio of free carboxyl groups to ester groups of 1:2 to a theoretical weight gain of 15% and a particle size of up to 1500 µm and of up to 2000 µm, and pellets coated with a mixed polymethacrylate coating of 5% of a methacrylic acid ethyl acrylate copolymer with a ratio of free carboxyl groups to ester groups of 1:1 and 95% of a copolymer of methacrylic acid and methyl methacrylate having a ratio of free carboxyl groups to ester groups of 1:2 to 15% weight gain, against time;

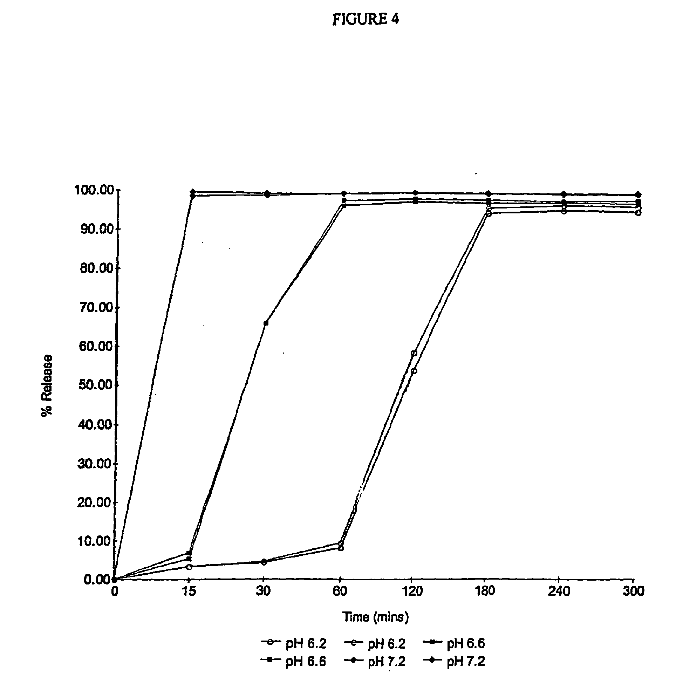

Figure 4 is a graph of percentage release (% Release), of paracetamol from pellets coated with a methacrylic acid copolymer of methacrylic acid and methyl methacrylate having a ratio of free carboxyl groups to ester groups of 1:2 to a theoretical weight gain of 20%, against time at a pH of 6.2, 6.6 and 7.2;

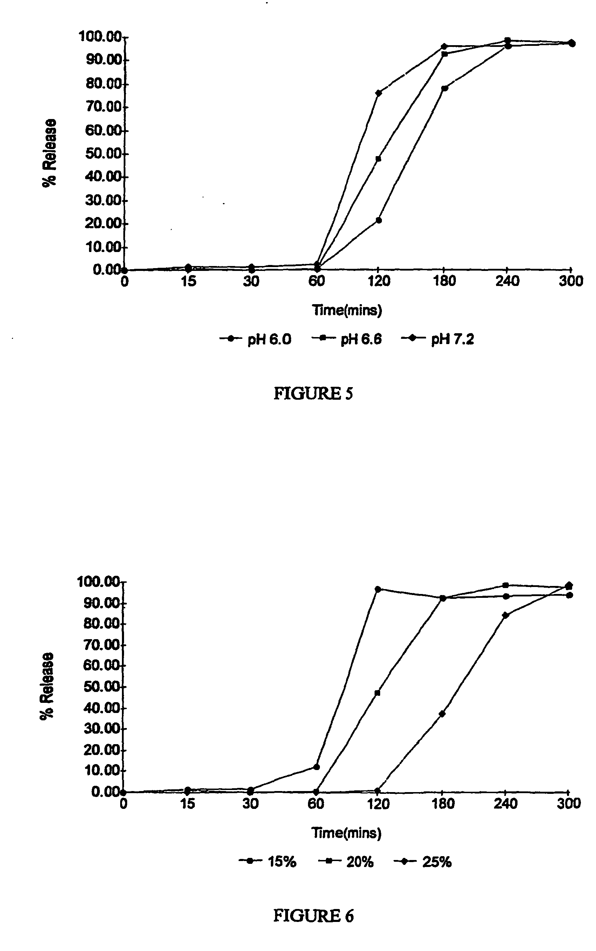

Figure 5 is a graph of percent release (% release), of metronidazole from pellets coated with a methacrylic acid copolymer of methacrylic acid and methyl methacrylate having a ratio of free carboxyl groups to ester groups of 1:2 to a theoretical weight gain of 20%, against time at a pH of 6.0, 6.6 and 7.2;
Figure 6 is a graph of percent release (% release), of metronidazole from pellets coated with a methacrylic acid copolymer of methacrylic acid and methyl methacrylate having a ratio of free carboxyl groups to ester groups of 1:2 to a theoretical weight gain of 15%, 20% and 25% against time at a pH of 6.6; and

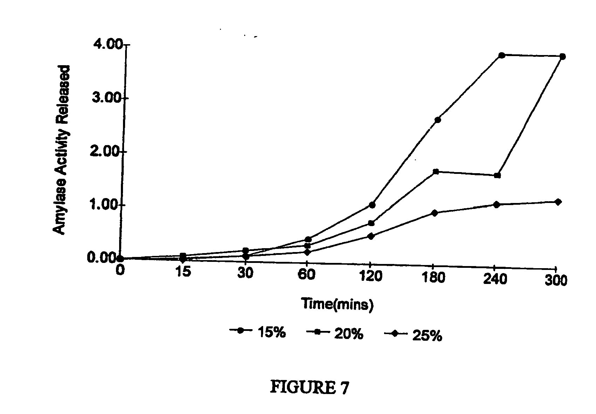

Figure 7 is a graph depicting how the activity of amylase, released from α-amylase pellets coated with a methacrylic acid copolymer of methacrylic acid and methyl methacrylate having a ratio of free carboxyl groups to ester groups of 1:2 to a theoretical weight gain of 15%, 20% and 25%, varies against time at a pH of 6.0.

### Example 1

Prednisolone metasulphobenzoate pellets were prepared by preparing a dry mix of 5 wt% prednisolone sodium metasulphobenzoate, 40 wt% microcrystalline cellulose (Avicel^{™} PH 101), 35 wt% lactose monohydrate (D80 200 Mesh) and 30 wt% croscarmellose sodium (Ac-Di-Sol^{™}) . Purified water (185 wt%) was added and the resulting mixture mixed for 10 minutes to form and extrudable paste which was then extruded from a 25 mm diameter bowel through a 1 mm diameter tube of about 5 mm length at a rate of about 100 mm/min, using a Nitro Fielder Type E140 extruder, and spheronised in a Nica System Spheroniser S700 on a 20 cm plate rotated at about 33 rpm. The pellets were then dried in a fluid bed granulator and screened to ensure the size of the particles was in the range 800 to 1500 µm.

The pellets were then spray coated with a methacrylic acid copolymer of methacrylic acid and methyl methacrylate having a ratio of free carboxyl groups to ester groups of 1:2 to provide three batches having a theoretical weight gain on coating (weight gain) of 5%, 15% and 25%.

The rate of release of prednisolone metasulphobenzoate from pellets having different thicknesses of coating and at a range of pH values was investigated.

### Example 2

The effect on the rate of release of prednisolone metasulphobenzoate from pellets having a coating of 5%, 15% and 25% weight gain, prepared as described in Example 1, was studied in a dissolution apparatus by stirring the pellets in a tribasic sodium phosphate medium at pH 6 and withdrawing samples at 15 minute intervals to measure, by HPLC, the amount of prednisolone metasulphobenzoate in solution. The results are shown in Figure 1.

As can be seen from Figure 1, increasing the thickness of the coating significantly decreases the rate of drug release. The 5% weight gain coated pellets provide complete (100%) drug release within 15 minutes. The 15% weight gain coated pellets, however, provided 50% drug release after about 45 minutes and 100% drug release after about 100 minutes and the 25% weight gain coated pellets provided 50% drug release after 120 minutes and 100% drug release after about 300 minutes.

It is particularly surprising that particles coated with the same pH dissolution dependent coating material, but at different thicknesses, provide drug release as such significantly different rates at the same pH.

### Example 3

The effect of pH on the rate of drug release from a coated pellet having a 15% weight gain coating prepared according to Example 1 was investigated. The pellets were subjected to drug release studies as described in Example 2 only using a pH of 6.0, 6.2, 6.6 and 7.2. Figure 2 illustrates the pH dependent nature of drug release from coated pellets having a 15% weight gain coating. As can be seen from Figure 2, at pH 6, complete drug release occurs at about 120 minutes, with 50% drug release at about 45 minutes. At higher pH, the rate of drug release increases until at pH 7.2, complete drug release occurs after about 30 minutes.

### Example 4

In order to investigate the effect of the precise coating composition on drug release, two batches of prednisolone metasulphobenzoate pellets having a 15% weight gain of either of two selected polymethacrylate coating materials were prepared by the method of Example 1. Pellets of the first batch were coated with a mixed polymethacrylate coating of 5% of a methacrylic acid ethyl acrylate copolymer with a ratio of free carboxyl groups to ester groups of 1:1 and 95% of a copolymer of methacrylic acid and methyl methacrylate having a ratio of free carboxyl groups to ester groups of 1:2 to 15% weight gain. Pellets of the second batch were coated with a copolymer of methacrylic acid and methyl methacrylate having a ratio of free carboxyl groups to ester groups of 1:2 to a weight gain of 15%.

The effect of coating composition on drug release was investigated by subjecting the two batches of pellets to a drug release study of the type described in Example 2. The results are illustrated in Figure 3.

As can be seen from Figure 3, batch 1, of which pellets are coated with a mixture of polymethacrylates - one which dissolves at pH 6.0 and one which dissolves at pH 5.5 - released drug at a greater rate than batch 2, of which pellets were coated with a polymethacrylate which dissolves at pH 6.0 to 7.0.

### Example 5

In order to investigate the effect of pellet size on drug release, prednisolone metasulphobenzoate pellets were prepared in two batches using the method of Example 1; the first batch having a diameter of up to 2000 µm and the second of up to 1500 µm and both having a coating of a copolymer of methacrylic acid and methyl methacrylate having a ratio of free carboxyl groups to ester groups of 1:2 to a weight gain of 15%. The pellets were subjected to a drug release study as per Example 4. The results of this are also shown in Figure 3.

As Figure 3 shows, increasing the pellet size resulted in a decrease in the rate of drug release. It is likely that this is because a larger pellet having a particular percentage weight gain of coating has a thicker coat than a smaller pellet with the same percentage weight gain of coating, because the ratio of surface area to weight is lower for the larger pellet.

### Example 6

Pellets containing paracetamol instead of prednisolone metasulphobenzoate were prepared to a method corresponding to that of Example 1 and coated with a copolymer of methacrylic acid and methyl methacrylate having a ratio of free carboxyl groups to ester groups of 1:2 to a weight gain of 20% and subjected to a drug release study similar to that of Example 3, only at a pH of 6.2, 6.6 and 7.2. The results are illustrated in Figure 4.

As can be seen from Figure 4, the rate of drug release appears to be pH dependent in that at pH 6.2, 50% of the drug is released at 120 minutes and complete drug release occurs at about 300 minutes whereas at pH 7.2, there is complete drug release within 15 minutes.

Accordingly, the ability to control the delay and rate of drug release is not limited to prednisolone metasulphobenzoate, but clearly can be more broadly applied.

### Example 7

Metronidazole pellets were prepared using the process as described in Example 1 with the exception that 20 wt % metronidazole was used in place of 5 wt % prednisolone metasulphobenzoate. The proportions of the remaining components were adjusted to 40 wt% microcrystalline cellulose (Avicel^{™} PH 101), 20 wt% lactose monohydrate and 20 wt% croscarmellose sodium (Ac-Di-Sol^{™}).

The metronidazole pellets were then spray coated with a methacrylic acid copolymer of methacrylic acid and methyl methacrylate having a ratio of free carboxyl groups to ester groups of 1:2 to provide coated pellets having a theoretical weight gain on coating (weight gain) of 20%.

The effect of pH on the rate of release of metronidazole from the metronidazole pellets was studied in a dissolution apparatus by stirring the pellets in a tribasic sodium phosphate medium at pH 6.0, pH 6.6 and then at pH 7.2 and withdrawing samples at 15 minute intervals to measure, by HPLC, the amount of metronidazole in solution. The results are shown in Figure 5.

As can be seen from Figure 5, at pH 6.0, complete drug release occurs at about 240 minutes, with 50% drug release at about 145 minutes. At higher pH, the rate of drug release increases until, at pH 7.2, complete drug release occurs after about 180 minutes.

### Example 8

Metronidazole pellets were prepared as described in Example 7. The pellets were then spray coated with a methacrylic acid copolymer of methacrylic acid and methyl methacrylate having a ratio of free carboxyl groups to ester groups of 1:2 to provide three batches having a theoretical weight gain on coating (weight gain) of 15%, 20% and 25%.

The effect of coating thickness on the rate of release of metronidazole from the coated metronidazole pellets was studied in a dissolution apparatus by stirring each batch of pellets in a tribasic sodium phosphate medium at pH 6.6 and withdrawing samples at 15 minute intervals to measure, by HPLC, the amount of metronidazole in solution. The results are shown in Figure 6.

As can be seen from Figure 6, increasing the thickness of the coating significantly decreases the rate of drug release. The 15% weight gain coated pellets provide complete (100%) drug release at about 120 minutes. The 20% weight gain coated pellets, however, provided 50% drug release after about 120 minutes and 100% drug release after about 240 minutes and the 25% weight gain coated pellets provided 50% drug release after about 200 minutes and 100% drug release after about 300 minutes.

### Example 9

α-Amylase pellets were prepared using the process as described in Example 1 with the exception that the α-amylase was dissolved in the granulation fluid (water). The proportions of the other components were 40 wt% microcrystalline cellulose (Avicel^{™} PH 101), 20 wt% lactose monohydrate and 40 wt% croscarmellose sodium (Ac-Di-Sol^{™}).

The pellets were then spray coated with a methacrylic acid copolymer of methacrylic acid and methyl methacrylate having a ratio of free carboxyl groups to ester groups of 1:2 to provide three batches having a theoretical weight gain on coating (weight gain) of 15%, 20% and 25%.

The effect of coating thickness on the rate of release of α-amylase from the coated α-amylase pellets at pH 6.0 was studied by colourimetry using the Sigma Enzymatic Assay of α-Amylase (EC 3.2.1.1) (Sigma-Aldrich Company Ltd., The Old Brickyard, New Road, Gillingham, Dorset, SP8 4XT, UK) and the results are shown in Figure 7.

As can be seen from Figure 7, as with the prednisolone metasulphobenzoate pellets and the metronidazole pellets, increasing the thickness of the coating significantly decreases the rate of drug release. The amount of release of the α-amylase is directly proportional to the activity observed. The 15% weight gain coated pellets provided maximum amylase activity at about 240 minutes. The 20% weight gain coated pellets, however, provided about 50% total activity after about 180 minutes and 100% total activity after about 300 minutes. The 25% weight gain coated pellets provided 25% total activity after about 180 minutes but, after 300 minutes, less than 50% total activity was observed.

The results of Examples 6 to 9 demonstrate that the invention is applicable to active compounds other than prednisolone metasulphobenzoate. The scope of application of the invention is therefore surprisingly broad.

It will be appreciated that the invention is not restricted to the details described above with reference to the preferred embodiments but that numerous modifications and variations can be made without departing from the scope of the invention as defined by the following claims.

## Claims

1. An oral pharmaceutical composition comprising two or more pluralities of pellets, said pellets comprising an active compound, wherein the pellets of each said plurality are coated with a different thickness, as determined by theoretical weight gain on coating, of a pH dissolution dependent coating material to those of the or each other plurality, wherein said coating material is applied onto the surface of the pellets, and whereby the active compound is released at different locations in the intestinal tract.

2. A composition as claimed in Claim 1, wherein the pellets of each plurality are coated with the same coating material as those of the or each other plurality.

3. A composition as claimed in Claim 1 or Claim 2, wherein the pH dissolution dependent coating material is a polymethacrylate material.

4. A composition as claimed in Claim 3, wherein the polymethacrylate material comprises a methacrylic acid copolymer.

5. A composition as claimed in Claim 3 or Claim 4, wherein the polymethacrylate material comprises a copolymer of methacrylic acid and methyl methacrylate.

6. A composition as claimed in any of Claims 3 to 5, wherein the polymethacrylate material is selected from a copolymer of methacrylic acid and methyl methacrylate having a ratio of free carboxyl groups to ester groups of about 1:2, a copolymer of methacrylic acid and methyl methacrylate having a ratio of free carboxyl groups to ester groups of about 1:1 or a mixture thereof.

7. A composition as claimed in any of Claims 3 to 6, wherein the pellets are coated with a methacrylic acid copolymer of methacrylic acid and methyl methacrylate having a ratio of free carboxyl groups to ester groups of about 1:2.

8. A composition as claimed in any of the preceding claims, wherein the pellets has a diameter in the range 800 to 1500µm.

9. A composition as claimed in any of the preceding claims, wherein the pellets are coated with the coating material to a theoretical weight gain on coating in the range 5% to 30%.

10. A composition as claimed in any of the preceding claims, wherein the pellets are coated with the coating material to a theoretical weight gain on coating in the range 10% to 25%.

11. A composition as claimed in any of the preceding claims, wherein the active compound is released at locations before and after the ileo-caecal valve.

12. A composition as claimed in any of the preceding claims, wherein the thickness of coating material coating pellets of each plurality of pellets is of increments chosen to provide a homogeneous release profile of the active compound along at least one selected portion of the intestinal tract.

13. A composition as claimed in any of the preceding claims, further comprising an enterically coated capsule within which the pluralities of pellets are contained.

14. A composition as claimed in any of the preceding claims, wherein there are two pluralities of pellets.

15. A composition as claimed in any of the preceding claims, wherein a first plurality of pellets is coated to provide a theoretical weight gain of 15% and a second plurality of particles is coated to provide a theoretical weight gain of 20%.

16. A composition as claimed in Claim 14 and Claim 15, wherein the first and second pluralities of pellets are present in a ratio of about 1:3.

17. A composition as claimed in any of the preceding claims, wherein the active compound is selected from the group consisting of peptides, polypeptides, proteins, interferons, TNF antagonists, hormones, cytokines, cytokine antagonists, analgesics, antipyretics, antibacterial agents, antiprotozoal agents, antiinflammatory agents, steroids, probiotics, prebiotics, antibiotics, bisphosphonates, cytotoxic agents, immunomodulators and antiparasitic agents.

18. A composition as claimed in any of the preceding claims, wherein the active compound is selected from the group consisting of erythropoietin, human growth hormone, metronidazole, clarithomycin, gentamycin, ciprofloxacin, rifabutin, 5-aminosalicylic acid, 4-aminosalicylic acid, balsalazide, α-amylase, paracetamol, metformin, prednisolone metasulphobenzoate, cyclophosphamide, cisplatin, vincristine, methotrexate, azathioprine, cyclosporin and albendazole.

19. A composition as claimed in any of the preceding claims, wherein the active compound is selected from the group consisting of prednisolone metasulphobenzoate, paracetamol, metronidazole and α-amylase.

20. Use of the coating thickness of a pH dissolution dependent coating material on pellets comprising an active compound, wherein the coating material is applied onto the surface of the pellets, to control the release profile of the active compound in the intestinal tract.

21. A use as claimed in Claim 20, wherein the coating material is a polymethacrylate material.

22. A use as claimed in Claim 21, wherein the polymethacrylate material comprises a methacrylic acid copolymer.

23. A use as claimed in Claim 21 or Claim 22, wherein the polymethacrylate material comprises a copolymer of methacrylic acid and methyl methacrylate.

24. A use as claimed in any of Claims 20 to 23, wherein the polymethacrylate material is selected from a copolymer of methacrylic acid and methyl methacrylate having a ratio of free carboxyl groups to ester groups of about 1:2, a copolymer of methacrylic acid and methyl methacrylate having a ratio of free carboxyl groups to ester groups of about 1:1 or a mixture thereof.

25. The use as claimed in any of Claims 20 to 24 wherein the active compound is selected from the group consisting of the compounds defined in any of Claims 17 to 19.

26. An oral composition as defined in any of Claims 1 to 19 for use in therapy or diagnosis practised on the human or animal body.

27. Use of a pH dissolution dependent coating material in the preparation of a medicament as defined in any of Claims 1 to 19 for the treatment of disorders of the intestinal tract.

28. A use as claimed in Claim 27, wherein the coating material is a polymethacrylate material.

29. A use as claimed in Claim 27 or Claim 28, wherein the coating material is a pH dissolution dependent polymethacrylate material.

30. A use as claimed in Claim 28 or 29 for the treatment of Crohn's disease.

## Patentansprüche

1. Orale pharmazeutische Zusammensetzung, umfassend zwei oder mehrere Vielzahlen von Pellets, wobei die Pellets einen Wirkstoff umfassen, wobei die Pellets einer jeder der Vielzahlen in einer unterschiedlichen Dicke, bestimmt durch theoretische Gewichtszunahme beim Überziehen, mit einem vom pH-Wert abhängig löslichen Überzugsmaterial überzogen sind im Vergleich zu jenen der bzw. jeder anderen Vielzahl, wobei das Überzugsmaterial auf die Oberfläche der Pellets aufgebracht wird und wobei der Wirkstoff an unterschiedlichen Orten im Verdauungstrakt freigesetzt wird.

2. Zusammensetzung wie in Anspruch 1 beansprucht, wobei die Pellets einer jeden Vielzahl mit dem gleichen Überzugsmaterial wie jene der bzw. jeder anderen Vielzahl überzogen sind.

3. Zusammensetzung wie in Anspruch 1 oder Anspruch 2 beansprucht, wobei das vom pH-Wert abhängig lösliche Überzugsmaterial Polymethacrylat-Material ist.

4. Zusammensetzung wie in Anspruch 3 beansprucht, wobei das Polymethacrylat-Material ein Methacrylsäure-Copolymer umfasst.

5. Zusammensetzung wie in Anspruch 3 oder Anspruch 4 beansprucht, wobei das Polymethacrylat-Material ein Copolymer von Methacrylsäure und Methylmethacrylat umfasst.

6. Zusammensetzung wie in einem der Ansprüche 3 bis 5 beansprucht, wobei das Polymethacrylat-Material ausgewählt ist aus einem Copolymer von Methacrylsäure und Methylmethacrylat mit einem Verhältnis von freien Carboxylgruppen zu Estergruppen von etwa 1:2, einem Copolymer von Methacrylsäure und Methylmethacrylat mit einem Verhältnis von freien Carboxylgruppen zu Estergruppen von etwa 1:1 oder einem Gemisch davon.

7. Zusammensetzung wie in einem der Ansprüche 3 bis 6 beansprucht, wobei die Pellets überzogen sind mit einem Methacrylsäure-Copolymer von Methacrylsäure und Methylmethacrylat mit einem Verhältnis von freien Carboxylgruppen zu Estergruppen von etwa 1:2.

8. Zusammensetzung wie in einem der vorhergehenden Ansprüche beansprucht, wobei die Pellets einen Durchmesser im Bereich von 800 bis 1500 µm aufweisen.

9. Zusammensetzung wie in einem der vorhergehenden Ansprüche beansprucht, wobei die Pellets überzogen sind mit dem Überzugsmaterial führend zu einer theoretischen Gewichtszunahme beim Überziehen im Bereich von 5 % bis 30 %.

10. Zusammensetzung wie in einem der vorhergehenden Ansprüche beansprucht, wobei die Pellets überzogen sind mit dem Überzugsmaterial führend zu einer theoretischen Gewichtszunahme beim Überziehen im Bereich von 10 % bis 25 %.

11. Zusammensetzung wie in einem der vorhergehenden Ansprüche beansprucht, wobei der Wirkstoff an Orten vor und nach der Ileozäkalklappe freigesetzt wird.

12. Zusammensetzung wie in einem der vorhergehenden Ansprüche beansprucht, wobei die Dicke des Überzugsmaterials, welches Pellets jeder der Vielzahlen von Pellets überzieht, aus Inkrementen gewählt ist, um ein homogenes Freisetzungsprofil des Wirkstoffes entlang mindestens eines ausgewählten Teils des Verdauungstrakts bereitzustellen.

13. Zusammensetzung wie in einem der vorhergehenden Ansprüche beansprucht, ferner umfassend eine magensaftresistent überzogene Kapsel, in welcher die Vielzahlen von Pellets enthalten sind.

14. Zusammensetzung wie in einem der vorhergehenden Ansprüche beansprucht, wobei zwei Vielzahlen von Pellets vorhanden sind.

15. Zusammensetzung wie in einem der vorhergehenden Ansprüche beansprucht, wobei eine erste Vielzahl von Pellets so überzogen ist, dass eine theoretische Gewichtszunahme von 15 % erzielt wird, und eine zweite Vielzahl von Teilchen so überzogen ist, dass eine theoretische Gewichtszunahme von 20 % erzielt wird.

16. Zusammensetzung wie in Anspruch 14 und Anspruch 15 beansprucht, wobei die ersten und zweiten Vielzahlen von Pellets in einem Verhältnis von etwa 1:3 vorhanden sind.

17. Zusammensetzung wie in einem der vorhergehenden Ansprüche beansprucht, wobei der Wirkstoff aus der Gruppe ausgewählt ist, welche aus Peptiden, Polypeptiden, Proteinen, Interferonen, TNF-Antagonisten, Hormonen, Zytokinen, Zytokinantagonisten, Analgetika, Antipyretika, antibakteriellen Mitteln, Antiprotozoika, entzündungshemmenden Mitteln, Steroiden, Probiotika, Präbiotika, Antibiotika, Bisphosphonaten, zytotoxischen Mitteln, Immunmodulatoren und antiparasitären Mitteln besteht.

18. Zusammensetzung wie in einem der vorhergehenden Ansprüche beansprucht, wobei der Wirkstoff aus der Gruppe ausgewählt ist, welche aus Erythropoietin, humanem Wachstumshormon, Metronidazol, Clarithomycin, Gentamycin, Ciprofloxacin, Rifabutin, 5-Aminosalicylsäure, 4-Aminosalicylsäure, Balsalazid, α-Amylase, Paracetamol, Metformin, Prednisolonmetasulfobenzoat, Cyclophosphamid, Cisplatin, Vincristin, Methotrexat, Azathioprin, Cyclosporin und Albendazol besteht.

19. Zusammensetzung wie in einem der vorhergehenden Ansprüche beansprucht, wobei der Wirkstoff aus der Gruppe ausgewählt ist, welche aus Prednisolonmetasulfobenzoat, Paracetamol, Metronidazol und α-Amylase besteht.

20. Verwendung der Überzugsdicke eines vom pH-Wert abhängig löslichen Überzugsmaterials auf Pellets, umfassend einen Wirkstoff, wobei das Überzugsmaterial auf die Oberfläche der Pellets aufgebracht wird, um das Freisetzungsprofil des Wirkstoffes im Verdauungstrakt zu steuern.

21. Verwendung wie in Anspruch 20 beansprucht, wobei das Überzugsmaterial ein Polymethacrylat-Material ist.

22. Verwendung wie in Anspruch 21 beansprucht, wobei das Polymethacrylat-Material ein Methacrylsäure-Copolymer umfasst.

23. Verwendung wie in Anspruch 21 oder Anspruch 22 beansprucht, wobei das Polymethacrylat-Material ein Copolymer von Methacrylsäure und Methylmethacrylat umfasst.

24. Verwendung wie in einem der Ansprüche 20 bis 23 beansprucht, wobei das Polymethacrylat-Material ausgewählt ist aus einem Copolymer von Methacrylsäure und Methylmethacrylat mit einem Verhältnis von freien Carboxylgruppen zu Estergruppen von etwa 1:2, einem Copolymer von Methacrylsäure und Methylmethacrylat mit einem Verhältnis von freien Carboxylgruppen zu Estergruppen von etwa 1:1 oder einem Gemisch davon.

25. Verwendung wie in einem der Ansprüche 20 bis 24 beansprucht, wobei der Wirkstoff aus der Gruppe ausgewählt ist, welche aus den Verbindungen besteht, die in einem der Ansprüche 17 bis 19 definiert sind.

26. Orale Zusammensetzung wie in einem der Ansprüche 1 bis 19 definiert zur Verwendung in der Therapie oder Diagnose, welche am menschlichen oder tierischen Körper praktiziert wird.

27. Verwendung eines vom pH-Wert abhängig löslichen Überzugsmaterials in der Herstellung eines Medikaments wie in einem der Ansprüche 1 bis 19 definiert zur Behandlung von Störungen des Verdauungstrakts.

28. Verwendung wie in Anspruch 27 beansprucht, wobei das Überzugsmaterial ein Polymethacrylat-Material ist.

29. Verwendung wie in Anspruch 27 oder Anspruch 28 beansprucht, wobei das Überzugsmaterial ein vom pH-Wert abhängig lösliches Polymethacrylat-Material ist.

30. Verwendung wie in Anspruch 28 oder 29 beansprucht zur Behandlung von Morbus Crohn.

## Revendications

1. Composition pharmaceutique orale comprenant deux ou plus de deux pluralités de granulés, lesdits granulés comprenant un composé actif, dans laquelle les granulés de chaque pluralité sont enrobés avec une épaisseur différente, telle que déterminée par le gain de poids théorique dû à l'enrobage, de celles de l'autre pluralité ou de chaque autres pluralité avec une substance d'enrobage dont la dissolution dépend du pH, dans laquelle ladite substance d'enrobage est appliquée sur la surface des granulés, moyennant quoi le composé actif est libéré à différents endroits dans le tractus intestinal.

2. Composition selon la revendication 1, dans laquelle les granulés de chaque pluralité sont enrobés avec une substance d'enrobage identique à celle de l'autre pluralité ou de chaque autre pluralité.

3. Composition selon la revendication 1 ou la revendication 2, dans laquelle la substance d'enrobage dont la dissolution dépend du pH est une substance à base de polyméthacrylate.

4. Composition selon la revendication 3, dans laquelle la substance à base de polyméthacrylate comprend un copolymère d'acide méthacrylique.

5. Composition selon la revendication 3 ou la revendication 4, dans laquelle la substance à base de polyméthacrylate comprend un copolymère d'acide méthacrylique et de méthacrylate de méthyle.

6. Composition selon l'une quelconque des revendications 3 à 5, dans laquelle la substance à base de polyméthacrylate est choisie parmi un copolymère d'acide méthacrylique et de méthacrylate de méthyle présentant un rapport entre les groupes carboxyles libres et les groupes esters d'environ 1 : 2, un copolymère d'acide méthacrylique et de méthacrylate de méthyle présentant un rapport entre les groupes carboxyles libres et les groupes esters d'environ 1 : 1 ou un de leurs mélanges.

7. Composition selon l'une quelconque des revendications 3 à 6, dans laquelle les granulés sont enrobés avec un copolymère d'acide méthacrylique composé d'acide méthacrylique et de méthacrylate de méthyle présentant un rapport entre les groupes carboxyles libres et les groupes esters d'environ 1 : 2.

8. Composition selon l'une quelconque des revendications précédentes, dans laquelle les granulés possèdent un diamètre situé dans la plage allant de 800 µm à 1500 µm.

9. Composition selon l'une quelconque des revendications précédentes, dans laquelle les granulés sont enrobés avec la substance d'enrobage pour donner un gain de poids théorique dû à l'enrobage situé dans la plage allant de 5 % à 30 %.

10. Composition selon l'une quelconque des revendications précédentes, dans laquelle les granulés sont enrobés avec la substance de d'enrobage pour donner un gain de poids théorique dû à l'enrobage situé dans la plage allant de 1 % à 25 %.

11. Composition selon l'une quelconque des revendications précédentes, dans laquelle le composé actif est libéré à des endroits avant et après la valvule iléo-caecale.

12. Composition selon l'une quelconque des revendications précédentes, dans laquelle l'épaisseur de la substance d'enrobage recouvrant les granulés de chaque pluralité de granulés est choisie pour augmenter de manière incrémentielle afin de donner un profil de libération homogène du composé actif le long d'au moins une partie sélectionnée du tractus intestinal.

13. Composition selon l'une quelconque des revendications précédentes, comprenant en outre une capsule à enrobage entérique à l'intérieur de laquelle les pluralités de granulés sont contenues.

14. Composition selon l'une quelconque des revendications précédentes, dans laquelle il y a deux pluralités de granulés.

15. Composition selon l'une quelconque des revendications précédentes, dans laquelle une première pluralité de granulés est enrobée pour donner un gain de poids théorique de 15 % et une seconde pluralité de particules est enrobée pour donner un gain de poids théorique de 20 %.

16. Composition selon la revendication 14 et la revendication 15, dans laquelle la première pluralité et la seconde pluralité de granulés sont présentes à un rapport d'environ 1 : 3.

17. Composition selon l'une quelconque des revendications précédentes, dans laquelle le composé actif est choisi dans le groupe constitué par les peptides, les polypeptides, les protéines, les interférons, les antagonistes de TNF, les hormones, les cytokines, les antagonistes de cytokines, les analgésiques, les antipyrétiques, les agents antibactériens, les agents anti-protozoaires, les agents anti-inflammatoires, les stéroïdes, les probiotiques, les prébiotiques, les antibiotiques, les bisphosphonates, les agents cytotoxiques, les immunomodulateurs et les agents antiparasitaires.

18. Composition selon l'une quelconque des revendications précédentes, dans laquelle le composé actif est choisi dans le groupe constitué par l'érythropoïétine, l'hormone de croissance humaine, le métronidazole, la clarithomycine, la gentamycine, la ciprofloxacine, la rifabutine, l'acide 5-aminosalicylique, l'acide 4-aminosalicylique, le balsalazide, l'α-amylase, le paracétamol, la metformine, le métasulfobenzoate de prednisolone, le cyclophosphamide, le cisplatine, la vincristine, le méthotréxate, l'azathioprine, la cyclosporine et l'albendazole.

19. Composition selon l'une quelconque des revendications précédentes, dans laquelle le composé actif est choisi dans le groupe constitué par le métasulfobenzoate de prednisolone, le paracétamol, le métronidazole et l'α-amylase.

20. Utilisation de l'épaisseur d'un enrobage d'une substance d'enrobage dont la dissolution dépend du pH sur des granulés comprenant un composé actif, dans laquelle la substance d'enrobage est appliquée sur la surface des granulés pour contrôler le profil de libération du composé actif dans le tractus intestinal.

21. Utilisation selon la revendication 20, dans laquelle la substance d'enrobage est une substance à base de polyméthacrylate.

22. Utilisation selon la revendication 21, dans laquelle la substance à base de polyméthacrylate comprend un copolymère d'acide méthacrylique.

23. Utilisation selon la revendication 21 ou la revendication 22, dans laquelle la substance à base de polyméthacrylate comprend un copolymère d'acide méthacrylique et de méthacrylate de méthyle.

24. Utilisation selon l'une quelconque des revendications 20 à 23, dans laquelle la substance à base de polyméthacrylate est choisie parmi un copolymère d'acide méthacrylique et de méthacrylate de méthyle présentant un rapport entre les groupes carboxyles libres et les groupes esters d'environ 1 : 2, un copolymère d'acide méthacrylique et de méthacrylate de méthyle présentant un rapport entre les groupes carboxyles libres et les groupes esters d'environ 1 : 1 ou un de leurs mélanges.

25. Utilisation selon l'une quelconque des revendications 20 à 24, dans laquelle le composé actif est choisi dans le groupe constitué par les composés définis dans l'une quelconque des revendications 17 à 19.

26. Composition orale selon l'une quelconque des revendications 1 à 19, pour un usage dans une thérapie ou un diagnostic pratiqué sur le corps d'un humain ou d'un animal.

27. Utilisation d'une substance d'enrobage dont la dissolution dépend du pH dans la préparation d'un médicament tel que défini dans l'une quelconque des revendications 1 à 19 pour le traitement des troubles du tractus intestinal.

28. Utilisation selon la revendication 27, dans laquelle la substance d'enrobage est une substance à base de polyméthacrylate.

29. Utilisation selon la revendication 27 ou la revendication 28, dans laquelle la substance d'enrobage est une substance à base de polyméthacrylate dont la dissolution dépend du pH.

30. Utilisation selon la revendication 28 ou 29, pour le traitement de la maladie de Crohn.
